# EUROPEAN PATENT SPECIFICATION

(11) **EP 0 585 722 B1**
(45) Date of publication and mention of the grant of the patent: **15.05.1996**
(21) Application number: 93113149.4
(22) Date of filing: 17.08.1993
(51) Int. Cl.: A61K 31/44

(54) **Benzimidazole derivatives as antimicrobal agent against Campylobacter pylon**
Benzimidazolverbindungen als Antibiotika gegen Campylobacter pylon
Dérivés de benzimidasoles comme agent antibiotique contre le campylobacter pylône

(30) Priority: 21.08.1992 JP 244042/92
(43) Date of publication of application: 09.03.1994
(73) Proprietor: Eisai Co., Ltd., Tokyo (JP)
(72) Inventor: Sato, Masaru, Koshigaya-shi, Saitama (JP); Souda, Shigeru, Ushiku-shi, Ibaraki (JP)
(74) Representative: Hansen, Bernd, Dr. Dipl.-Chem.

(56) References cited:
- EP-A- 0 268 956
- EP-A- 0 382 489
- Dialog Information Services File 187, FDC Reports, 1987-93, Accession-no. 92407, The Pink Sheet, vol. 55, issue 30, 26.7.93: 'LYS-PRO modified insulin 1994 NDA filing a possibility, Lilly indicates; Lovan application has been switched to FDA Endocrine & Metabolism Division'
- EUR. J. CLIN. MICROBIOL. INFECT. DIS., vol. 10, no. 2, 1991, pages 92-93; S. SUERBAUM ET AL.: 'Antibacterial activity of pantoprazole and omeprazole against Helicobacter pylori'

## Description

The invention relates to an antimicrobial agent, an antibiotic, anti-bacterial medicine which prevents, improves and therapeutically treats a disease caused by Campylobacter pylori, called hereinafter C. pylori. This organism is a microaerophilic, gram-negative spirillar rod and it is thought to cause infectious diseases such as diarrhea and food poisoning. Some investigators have also proposed that Campylobacter pylori may participate in the pathogenesis of gastritis and in particular the recurrent form thereof. In 1989, it was proposed to rename C. pylori to Helicobacter pylori based on further research in the fields of bacteriology and bacterial taxology. Therefore, C. pylori is also called H. pylori.

### Statement of prior art

In the state of the art, many substances have been used for prevention, treatment or cure of stomach and duodenum diseases such as gastritis or peptic ulcers, gastric ulcers and duodenal ulcers. Especially, histamine H₂-receptor antagonists such as cimetidine or ranitidine have been widely used. Histamine H₂-receptor antagonists quickly improve a damaged stomach and duodenal mucous membrane, and they reduce subjective symptoms such as stomach pain immediately. However, the problem remains that these diseases will recur very often after they have been healed and the medication has been ceased.

It is reported in the Medical Journal of Australia, volume 142, pages 436-439, 1985 and American Journal of Gastroenterology, volume 82, pages 192-199, 1987 that healthy volunteers to whom C. pylori was administered orally, started to show symptoms of acute gastritis. Gastroenterology, volume 94, pages 33-40, 1988 reported that the injured gastric tissue was found to improve when C. pylori was eradicated by administration of antibiotic substances. Thus, it was shown that some forms of gastritis are actually caused by C. pylori and can be effectively treated by eliminating C. pylori.

In the state of the art, medicines which can eradicate C. pylori include antibiotics such as ampicillin, cefalexin, ofloxacin, minocycline and roxithromycin and medicaments for gastritis such as plaunotol, sofalcone, benexate hydrochloride and colloidal bismuth subcitrate. Among these substances which can clear C. pylori, prior medicines for gastritis provide an eradication activity which is too weak in a usual dosage of administration. The antibiotics mentioned above have a good efficacy against C. pylori, but some side-effects such as allergy or diarrhea will often occur, and then, after long term administration, they sometimes cause severe irreversible adverse reactions such as hematological disorders or gastrointestinal disorders. And growth of resistant bacteria easily occurs. From a clinical point of view, a good medicine is desired for reliable prevention and therapy and for a long term medication of gastritis, in particular the recurrent form of gastritis, with safety. There is continuing research for a medicine which can eradicate C. pylori in a usual dosage of administration with safety in the long term medication.

JP-A 2-209809, corresponding to US 5 093 342 discloses that 5-Methoxy-2-[(4-methoxy-3,5-dimethyl-2-pyridinyl)methylsulfinyl]-1H-benzimidazole, called omeprazole, or a salt thereof, being an acid secretion-inhibiting medicine for gastritis etc., has antibiotic activity against C. pylori 8005, which is one particular clinical isolate of Campylobacter pylori. On the other hand, Scand. J. Gastroenterol., 24 (1989), 12-15, and Scand. J. Gastroenterol. 24 (suppl. 167), (1989), 49-54, disclose tests to demonstrate that omeprazole shows no antimicrobial acitivity on Campylobacter pylori in vivo.

JP-A 3-173817, corresponding to US 5 013 743 discloses the compounds (1) to (14) shown below exhibiting an antibiotic activity against the standard strains C. pylori NCTC-11916 and NCTC-11637, and the clinical isolates C. pylori PCL-56, CPY-0011-1, KS-13, CLO-1 and CLO-6.
(1) 2-[{3-Methyl-4-(2,2,2-trifluoroethoxy)pyrid-2-yl}methylsulfinyl]-1H-benzimidazole
(2) 2-(3,5-Dimethyl-4-methoxypyrid-2-yl)methylsulfinyl-5-methoxy-1H-benzimidazole
(3) 2-[{3-Methyl-4-(2,2,3,3,3- pentafluoropropoxy)pyrid-2-yl-methylsulfinyl]-1H-benzimidazole
(4) 2-[{3-Methyl-4-(2,2,3,3- tetrafluoropropoxy)pyrid-2-yl}methylsulfinyl]-1H-benzimidazole
(5) 2-[{3-Methyl-4-(2,2,3,3,4,4- hexafluorobutoxy)pyrid-2-yl}methylsulfinyl]-1H-benzimidazole
(6) 2-[{3-Methyl-4-(2,2,2-trifluoroethoxy)pyrid-2-yl}methylthio]-1H-benzimidazole
(7) 2-[{(4-Isobutoxy)pyrid-2-yl}methylthio]-1H-benzimidazole
(8) 2-[{(4-Isobutoxy)pyrid-2-yl}methylthio]-5-trifluoromethyl-1H-benzimidazole
(9) 2-[{(4-Isopropoxy)pyrid-2-yl}methylthio]-5-trifluoromethyl-1H-benzimidazole
(10) 2-[[{4-(2'-Propenyl)oxy}pyrid-2-yl]methylthio]-5-trifluoromethyl-1H-benzimidazole
(11) 2-[{(4-Propargyl)oxypyrid-2-yl}methylthio]-5-trifluoromethyl-1H-benzimidazole
(12) 2-[{3-Methyl-4-(2,2,3,3-tetrafluoropropoxy)pyrid-2-yl}methylthio]-1H-benzimidazole
(13) 2-[{3-Methyl-4-(2,2,3,3,3-pentafluoropropoxy)pyrid-2-yl}methylthio]-1H-benzimidazole
(14) 2-[{3-Methyl-4-(2,2,3,3,4,4-hexafluorobutoxy)pyrid-2-yl}methylthio]-1H-benzimidazole
JP-A 3-48680 discloses the below shown compounds being antibiotic against C. pylori, failing to show tested strains.
(1) 2-[3-Methyl-4-(1-methyl-2-piperidyl)methoxy-2-pyridyl]methylthio-1H-benzimidazole
(2) 2-[3-Methyl-4-(3-morpholinopropoxy)-2-pyridyl]methylthio-1H-benzimidazole
(3) 2-[3-Methyl-4-(2-piperidinoethoxy)-2-pyridyl]methylthio-1H-benzimidazole
(4) 2-[3-Methyl-4-{2-(2-oxo-1-pyrrolidinyl)ethoxy}-2-pyridyl]methylthio-1H-benzimidazole
(5) 2-[3-Methyl-4-(2-morpholinoethoxy)-2-pyridyl]methylthio-1H-benzimidazole
(6) 2-[3-Methyl-4-(3-piperidinopropoxy)-2-pyridyl]methylthio-1H-benzimidazole
(7) 5-Methoxy-2-[3-methyl-4-(2-morpholinoethoxy)-2-pyridyl]methylthio-1H-benzimidazole
(8) 5-Methoxy-2-[3-methyl-4-{2-(N-benzyl-N-methylamino)ethoxy}-2-pyridyl]methylthio-1H-benzimidazole
(9) 2-[3-Methyl-4-[2-{N-methyl-N-(2-phenylethyl)amino}ethoxy]-2-pyridyl]methylthio-1H-benzimidazole
(10) 2-[3-Methyl-4-[2-(N-methyl-N-(3-phenylpropyl)amino}ethoxy]-2-pyridyl]methylthio-1H-benzimidazole
(11) 2-[3-Methyl-4-{2-(N-benzyl-N-ethylamino)ethoxy}-2-pyridyl]methylthio-1H-benzimidazole
(12) 2-[3-Methyl-4-{2-(N-benzyl-N-propylamino)ethoxy}-2-pyridyl]methylthio-1H-benzimidazole
(13) 2-[3-Methyl-4-[2-{N-methyl-N-(4-methylbenzyl)amino}ethoxy]-2-pyridyl]methylthio-1H-benzimidazole
(14) 2-[3-Methyl-4-[2-{N-(4-chlorobenzyl)-N-methylamino}ethoxy]-2-pyridyl]methylthio-1H-benzimidazole
(15) 2-[3-Methyl-4-[2-{N-(4-bromobenzyl)-N-methylamino}ethoxy]-2-pyridyl]methylthio-1H-benzimidazole
(16) 2-[3-Methyl-4-{2-(1,2,3,4-tetrahydroisoquinoline-2-yl)ethoxy}-2-pyridyl]methylthio-1H-benzimidazole
(17) 1/2 magnesium salt of 2-[3-methyl-4-{2-(N-benzyl-N-methylamino)ethoxy}-2-pyridyl]methylsulfinyl-1H-benzimidazole
(18) 1/2 magnesium salt of 2-[3-Methyl-4-[2-{N-methyl-N-(4-methylbenzyl)amino}ethoxy]-2-pyridyl]methylsulfinyl-1H-benzimidazole
(19) 1/2 magnesium salt of 2-[3-Methyl-4-[2-{N-(4-bromobenzyl)-N-methylamino}ethoxy]-2-pyridyl]methylsulfinyl-1H-benzimidazole
(20) 2-[3-Methyl-4-[2-(1,2,3,4-tetrahydroisoquinoline-2-yl)ethoxy]-2-pyridyl]methylsulfinyl-1H-benzimidazole
(21) 2-[3-Methyl-4-[2-(N-benzyl-N-methylamino)ethoxy]-2-pyridyl]methylsulfonyl-1H-benzimidazole
(22) 2-[3-Methyl-4-[2-(N-methyl-N-(4-methylbenzyl)amino)ethoxy]-2-pyridyl]methylsulfonyl-1H-benzimidazole
In the medication of diseases caused by C. pylori, it is necessary to have an excellent activity against a broad spectrum of C. pylori including standard strains and clinical isolates. The antibiotics effective against C. pylori have a minimum inhibitory concentration called MIC (µg/ml) in the range of about 1 µg/ml or less. Therefore, whether a compound has a MIC in that range or not is a criterion to evaluate its clinical efficacy against C. pylori.

Under the above shown point of view, omeprazole disclosed in JP-A 2-209809 and JP-A 3-173817 has no sufficient antibiotic activity. Among the other 14 compounds shown in JP-A 3-173817, the compounds (12), (13) and (14) are the only antibiotics in respect of activity against all the tested strains. JP-A 3-48680 shows that the compounds have an MIC of 1 microgram per ml or less to only one tested strain. It is not expected from the listed data that they will have a wide antibiotic activity against many strains.

2-[(4-(3-Methoxypropoxy)-3-methylpyridine-2-yl)-methylsulfinyl]-1H-benzimidazole (I; n=1) and 2-[(4-(3-Methoxypropoxy)-3-methylpyridine-2-yl)-methylthio]-1H-benzimidazole (I; n=0) are known from JP-A 1-6270, corresponding to EP-A 268 956, as acid secretion inhibitors having an anti-ulcer activity. They can be prepared according to Examples 32 and 31, respectively, disclosed in JP-A-1-6270. However, the antimicrobial activity of these compounds is not referred to.

### Disclosure of the Invention

The inventors made investigations with 2-[(4-(3-Methoxypropoxy)-3-methylpyridine-2-yl)-methylsulfinyl]-1H-benzimidazole (I; n=1) and 2-[(4-(3-Methoxypropoxy)-3-methylpyridine-2-yl)-methylthio]-1H-benzimidazole (I; n=0) and surprisingly found them to have the same C. pylori-eradicating activity as an antibiotic substance. This is how the inventors have reached the invention. The two compounds of the invention have the following chemical structure: 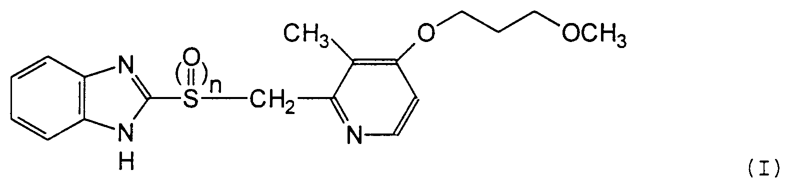
wherein n is 0 or 1, respectively.

The invention provides the use of a pharmacologically effective amount of a compound having the above formula (I) wherein n is 0 or 1, or a pharmacologically acceptable salt thereof for the preparation of a medicament for preventing, improving or treating a disease caused by Campylobacter pylori in a mammal suffering from such a disease.

It is preferable that the mammal is a human being and the disease is peptic ulcer or gastritis, in particular the recurrent form thereof. The disease may also be an infectious disease caused by Campylobacter pylori such as diarrhea and food poisoning.

Moreover, the invention provides the use of a compound having the above formula (I) wherein n is 0 or 1, or a pharmacologically acceptable salt thereof for the preparation of a medicament for eradicating C. pylori from the mucous membrane of the stomach of a patient suffering from a gastric ulcer, duodenal ulcer or gastritis if the medicament is administered in a pharmacologically effective amount to the patient;
the use of a pharmacologically effective amount of a compound having the above formula (I) wherein n is 0 or 1, or a pharmacologically acceptable salt thereof for the preparation of a medicament for preventing recurrence of a disease caused by C. pylori in a mammal suffering from said disease;
and the use of a pharmacologically effective amount of a compound having the above formula (I) wherein n is 0 or 1 or a pharmacologically acceptable salt thereof for the preparation of a medicament for restraining the adverse activity of C. pylori to cause a peptic disease in a mammal.

The invention further provides an antimicrobial medicine or agent comprising a compound having the formula (I) wherein n is 0 or 1, or a pharmaceutically acceptable salt thereof to prevent, improve or treat an ulcer and/or gastritis. In the invention, the pharmacologically acceptable salt of the compound (I) wherein n is 0 or 1, includes an addition salt thereof with an inorganic acid such as hydrochloric acid, sulfuric acid, hydrobromic acid, hydroiodic acid, nitric acid, perchloric acid and phosphoric acid, an addition salt thereof with an organic acid such as citric acid, maleic acid, fumaric acid, succinic acid, methanesulfonic acid, benzenesulfonic acid and p-toluenesulfonic acid, and a salt thereof with a metal such as sodium, potassium, calcium and magnesium. The sodium salts, in particular those having the following formula (II) are most preferable for administration: 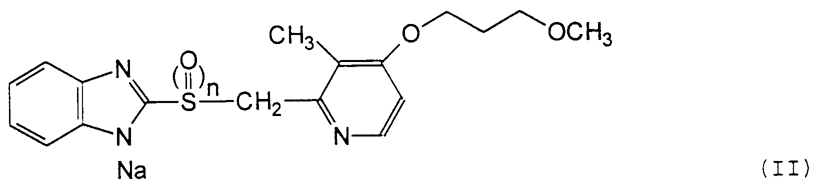
wherein n is 0 or 1.

The invention provides a medicine which is effective for the C. pylori eradication having the same effect as antibiotics. The compounds of the invention can be administered for a long time with safety and without side effects. They are pharmacologically effective and clinically useful against diseases caused by Campylobacter pylori, in particular peptic ulcer or (recurring) gastritis.

The compounds of the invention have the following physical properties:
Physical properties of 2-[(4-(3-Methoxypropoxy)-3-methylpyridine-2-yl)-methylsulfinyl]-1H-benzimidazole (I; n=1):
molecular formula : C₁₈H₂₀N₃O₃S
molecular weight : 358.44
appearance : white crystalline
Physical properties of 2-[(4-(3-Methoxypropoxy)-3-methylpyridine-2-yl)-methylthio]-1H-benzimidazole (I; n=0):
molecular formula : C₁₈H₂₀N₃O₂S
molecular weight : 342.44
appearance: pale yellow crystalline

### Pharmacological Test 1:

Standard strains and clinical isolates of C. pylori derived from the mucous membrane of the stomach were used and determined in vitro according to the agar dilution method by Nihon Kagaku Ryoho Gakkai (English name: Japan Society of Chemotherapy).

The sodium salt of 2-[(4-(3-Methoxypropoxy)-3-methylpyridine-2-yl)-methylsulfinyl]-1H-benzimidazole (I; n=1) was dissolved in sterilized water. 2-[(4-(3-Methoxypropoxy)-3-methylpyridine-2-yl)-methylthio]-1H-benzimidazole (I; n=0) and, as controls, omeprazole, 5-Methoxy-2-[(4-methoxy-3,5-dimethyl-2- pyridinyl)methylthio]-1H-benzimidazole and 2-[(3-Methyl-4-(2,2,2-trifluoroethoxy)pyrid-2-yl)methylthio]-1H-benzimidazole were dissolved separately in a 1% dimethylsulfoxide solution. As antibiotic control substances, roxithromycin as a macrolide, ampicillin as a penicilline and ofloxacin as a quinolone were dissolved in a buffer solution of acetic acid having a pH of 5.0, sterilized water and an 1N aqueous solution of NaOH, respectively. Test plates were prepared by adding 7% horse blood to Brucella agar named by BBL Microbiology Systems (tradename), being available from Bector Dickson and Company. The incubation was conducted at 37°C at a pH of 7.0 for 3 days under microaerophilic conditions using Canpipack (tradename) being available from Bector Dickson and Company. MIC was determined in units of microgram per ml. Test results are shown in Table 1. NCTC11637 and NCTC11916 are standard strains. The other references indicate clinical isolates.

The test result is shown in terms of the antibiotic activity (MIC) in units of microgram/ml, obtained in vitro against C. pylori. The tested compounds are:
(1) sodium salt of 2-[4-(3-Methoxypropoxy)-3-methylpyridine-2-yl]methylsulfinyl-1H-benzimidazole,
(2) 2-[4-(3-Methoxypropoxy)-3- methylpyridine-2-yl]methylthlo-1H-benzimidazole,
(3) 5-Methoxy-2-(4-methoxy-3,5-dimethyl-2-pyridine-2-yl)methylsulfinyl-1H-benzimidazole (omeprazole),
(4) 5-Methoxy-2-(4-methoxy-3,5-dimethyl-2-pyridinyl)methylthio-1H-benzimidazole,
(5) 2-[3-Methyl-4-(2,2,2-trifluoroethoxy)pyridine-2-yl]methylthio-1H-benzlmidazole,
(6) roxithromytin (RXM),
(7) ampicillin (ABPC),
(8) ofloxacin (OFLX).

**Table 1**

| Tested strain | Compound | | | | | | | |
|---|---|---|---|---|---|---|---|---|
| | (1) | (2) | (3) | (4) | (5) | (6) | (7) | (8) |
| NCTC11637 | 3.13 | 0.8 | 50 | 25 | 25 | 0.4 | 0.1 | 0.8 |
| NCTC11916 | 3.13 | 0.2 | 25 | 12.5 | 12.5 | 0.1 | 0.05 | 0.8 |
| EI-2 | 3.13 | 0.4 | 25 | 25 | 25 | 0.2 | 0.4 | 0.8 |
| EI-5 | 3.13 | 0.2 | 50 | 25 | 12.5 | 0.2 | 0.4 | 0.8 |
| EI-36 | 6.25 | 0.4 | 25 | 12.5 | 12.5 | 0.2 | 0.2 | 0.8 |
| EI-46 | 3.13 | 0.4 | 25 | 12.5 | 12.5 | >100 | 0.05 | 3.13 |
| EI-391 | 3.13 | 1.56 | 50 | 25 | 12.5 | 50 | 0.1 | 25 |
| EI-393 | 3.13 | 1.56 | 50 | 25 | 25 | 0.2 | 0.1 | 0.8 |
| EI-397 | 3.13 | 1.56 | 50 | 25 | 25 | 0.2 | 0.1 | 0.8 |
| EI-429 | 1.56 | 1.56 | 25 | 25 | 25 | 0.2 | 0.2 | 0.8 |
| EI-467 | 1.56 | 1.56 | 50 | 12.5 | 12.5 | 0.2 | 0.05 | 0.4 |
| EI-612 | 0.4 | 0.8 | 25 | 12.5 | 6.25 | 0.2 | 0.05 | 0.8 |
| EI-925 | 3.13 | 0.4 | 25 | 12.5 | 12.5 | 0.2 | 0.2 | 0.4 |
| EI-930 | 3.13 | 0.4 | 25 | 12.5 | 12.5 | 0.4 | 0.4 | 0.8 |
| EI-933 | 3.13 | 0.8 | 25 | 12.5 | 12.5 | 0.1 | 0.05 | 0.4 |

It is noted from the test results that the compounds of the invention are superior to known compounds such as omeprazole derivatives disclosed in JP-A 2-209809 and lansoprazole derivatives (6) disclosed in JP-A 3-173817. The compounds of the invention evidently provide an equivalent C. pylori eradicating activity to antibiotics.

### Pharmacological Test 2:

The following compounds (1) to (11) were tested in the same way as shown in Pharmacological Test 1 and the results are shown in Table 2.
(1) sodium salt of 2-[4-(3-Methoxypropoxy)-3-methylpyridine-2-yl]methylsulfinyl-1H-benzimidazole,
(2) 2-[4-(3-Methoxypropoxy)-3-methylpyridine-2-yl)methylthio-1H-benzimidazole,
(3) 5-Methoxy-2-(4-methoxy-3,5-dimethylpyridine-2-yl)methylsulfinyl-1H-benzimidazole (omeprazole),
(4) 5-Methoxy-2-(4-methoxy-3,5-dimethylpyridine-2-yl)methylthio-1H-benzimidazole (thioether of omeprazole),
(5) 2-[3-Methyl-4-(2,2,2-trifluoroethoxy)pyridine-2-yl]methylthio-1H-benzimidazole (thioether of lansoprazole),
(6) roxithromycin (RXM),
(7) ampicillin (ABPC),
(8) ofloxacin (OFLX),
(9) 2-[3-Methyl-4-(2,2,2-trifluoroethoxy)pyridine-2-yl]methylsulfinyl-1H-benzimidazole (lansoprazole),
(10) 2-[3-Methyl-4-(2-morpholinoethoxy)pyridine-2-yl]methylthio-1H-benzimidazole (disclosed in JP-A 3-48680),
(11) 2-[3-Methyl-4-(2,2,3,3-tetrafluoropropoxy)pyridine-2-yl]methylsulfinyl-1H-benzimidazole (disclosed in US-A 5 013 743)

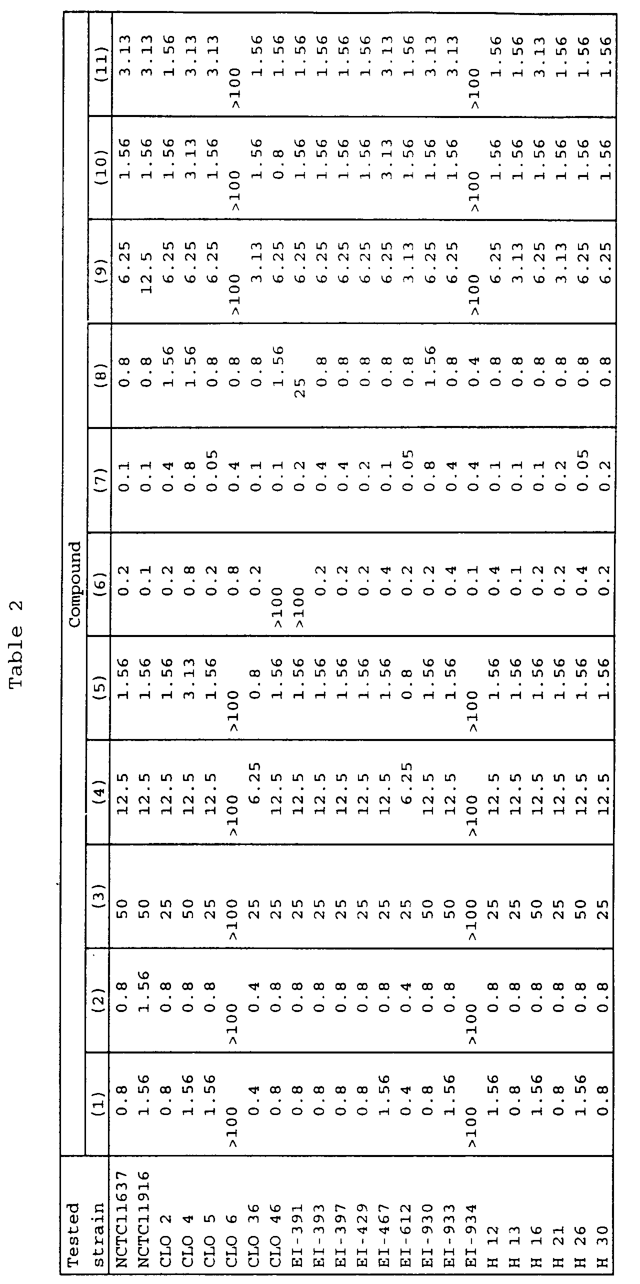

It is noted from the data of Table 1 and Table 2 that the compounds of formula (I) according to the invention wherein n is 0 or 1, are superior to omeprazole, 2-[3-methyl-4-(2,2,2-trifluoroethoxy)-pyridine-2-yl]-methylthio-1H-benzimidazole and 2-[3-methyl-4-(2,2,2-trifluoroethoxy)-3-pyrido-2-yl]-methylsulfinyl-1H-benzimidazole and at the same time they have almost the same C. pylori-eradicating activity as antibiotic substances.

Since the compounds of the invention are not antibiotic substances in the usual sense, they are effective against resistant bacteria such as EI-46 and EI-39l of the clinical isolated strains shown in Table 1. This is the reason why they provide an eradicating activity to a wider variety of bacteria than conventional antibiotic substances. They can be administered to a mammal and the human being for a long time. They selectively eradicate C. pylori, not inhibiting the growth of other gram positive bacteria and gram negative bacteria even at a concentration being as high as 100 µg per ml or above. This shows the fact that they can selectively be antibiotic against C. pylori.

### Acute Toxicological Test:

The compound (I; n=1) of the invention was administered intravenously or orally one time, with a carrier of saline, to Slc:SD rats and Slc:ICR mice, each being 7 or 8 weeks old, in a group consisting of five males and five females, to determine LD₅₀ values. Results are shown in Table 3.

The acute toxicity of the compound (I; n=1) is shown in terms of LD₅₀ in units of mg/kg.

**Table 3**

| administration route | rats | | mice | |
|---|---|---|---|---|
| | male | female | male | female |
| intravenous | 157 mg/kg | 152 mg/kg | 220 mg/kg | 237 mg/kg |
| oral | 1447 mg/kg | 1332 mg/kg | 1206 mg/kg | 1012 mg/kg |

The obtained LD₅₀ values are 5000 times as much as a clinical dosage of oral administration and for this reason the compounds of the invention are found to be greatly safe, that is, providing no toxicological influence.

### Technological Advances of the Invention:

It is evident from the above test data that the compounds of the invention are as antibiotically active against C. pylori as conventional antibiotic substances and therefore can work pharmacologically to prevent, improve and therapeutically treat diseases caused by C. pylori such as peptic ulcer or gastritis and some infectious diseases. They can prevent, improve and therapeutically treat these diseases, particularly recurring gastritis caused by C. pylori, with safety. They can be administered continuously for a long term, without combination of another antibiotic substance. The compounds can be used as an antibiotic medicine.

### Application of the Invention:

The compounds of the invention can be administered, without any limitation, in normal ways. Which way and what amount is to be used for medication depends on the condition of the patient, the kind of ulcer and/or gastritis, the degree or extent of the disease, age of the patient and functions of heart, liver and kidney. It is preferable in the invention that the compounds of the invention are dosed orally in an amount of 0.01 to 100 mg per day, more preferably 0.1 to 80 mg, further preferably 0.1 to 60 mg, most preferably 5 to 40 mg. The oral medication can be conducted in the form of powder, granule, tablet or capsule. They can be prepared with a normally used carrier according to prior art methods, preferably according to the methods shown in JP-A 1-290628 and JP-A 2-22225, corresponding to US 5 035 899. The preparation may further include therein a forming filler, a binder, a disintegrating agent, a lubricant, a colorant, a corrigent, a taste-improver and/or a smell-improver.

The filler includes lactose, corn starch, white sucrose, glucose, mannitol, sorbitol, crystalline cellulose and silicon dioxide ; the binder includes polyvinyl alcohol, polyvinyl ether, ethylcellulose, methylcellulose, acacia, tragacanth, gelatin, shellac, hydroxypropylcellulose, hydroxypropylstarch and polyvinylpyrrolidone ; the disintegrating agent includes starch, agar, gelatin powder, crystalline cellulose, calcium carbonate, sodium hydrogencarbonate, calcium citrate, dextrin, carboxymethyl cellulose, calcium and pectin; the lubricant includes magnesium stearate, talc, polyethylene glycol, silica and hardened vegetable oils ; the colorant may be any one which is permitted for drugs ; the corrigent includes cacao powder, mentha herb, aromatic powder, mentha oil, borneol and powdered cinnamon bark. Of course, these tablets and granules may be, if necessary, coated with sugar, gelatin or the like.

Examples of preparations of the medicines of the invention, including orally dosed preparations and those with a enteric coating including the compound (I) wherein n is 0 or 1, of the invention are shown below. These do not mean any limitation to the invention.

### Example 1

Tablets including the compound (I; n=1): 2-[4-(3-Methoxypropoxy)-3-methylpyridine-2-yl]-methylsulfinyl-1H-benzimidazole were prepared by the below shown prescription.

2-[4-(3-Methoxypropoxy)-3-methylpyridine-2-yl]-methylsulfinyl-1H-benzimidazole, mannitol and magnesium oxide were mixed with each other, hydroxypropylcellulose dissolved in ethanol was added to the mixture, the resultant mixture was granulated, dried and classified with a sieve of 28 mesh according to ASTM, the product being called (A). Then crystalline cellulose and corn starch were mixed with the product (A), hydroxypropylcellulose dissolved in water was added to the mixture, the resultant mixture was granulated, dried and classified with a sieve of 28 mesh, the product being called (B). The products (A) and (B), calcium salt of carboxymethylcellulose, talc and magnesium stearate were mixed with each other and the mixture was formed into plain tablets with a single shot tablet-formulating machine, being available from Okada Seiko-sha.

Ingredients of 1 plain tablet weighing 120.2 mg are shown below in units of mg:

| | |
|---|---|
| 2-[4-(3-Methoxypropoxy)-3-methylpyridine-2-yl]-methylsulfinyl-1H-benzimidazole | 5.0 |
| mannitol | 45.3 |
| magnesium oxide | 40.0 |
| hydroxypropylcellulose | 2.5 |
| crystalline cellulose | 10.0 |
| corn starch | 10.0 |
| carboxymethylcellulose calcium | 5.0 |
| talc | 2.0 |
| magnesium stearate | 0.2 |

The obtained plain tablets were coated with an enteric coating to obtain tablets.

### Example 2

Tablets including 2-[4-(3-Methoxypropoxy)-3-methylpyridine-2-yl]-methylthio-1H-benzimidazole (I; n=0) were prepared by the below shown prescription.

2-[4-(3-Methoxypropoxy)-3-methylpyridine-2-yl]-methylthio-1H-benzimidazole and mannitol were mixed with each other, hydroxypropylcellulose dissolved in ethanol was added to the mixture, the resultant mixture was granulated, dried and classified with a sieve of 28 mesh according to ASTM, the product being called (A). Then (A), crystalline cellulose, corn starch, calcium salt of carboxymethylcellulose, talc and magnesium stearate were mixed with each other and the mixture was formed into plain tablets with a single shot tablet-formulating machine, being available from Okada Seiko-sha.

Ingredients of 1 plain tablet weighing 99.7 mg are shown below in units of mg:

| | |
|---|---|
| 2-[4-(3-Methoxypropoxy)-3-methylpyridine-2-yl]-methylthio-1H-benzimidazole | 5.0 |
| mannitol | 65.3 |
| hydroxypropylcellulose | 2.5 |
| crystalline cellulose | 10.0 |
| corn starch | 10.0 |
| carboxymethylcellulose calcium | 5.0 |
| talc | 2.0 |
| magnesium stearate | 0.2 |

The obtained plain tablets were coated with an enteric coating to obtain tablets.

## Claims

1. The use of a pharmacologically effective amount of a compound having the following formula (I) 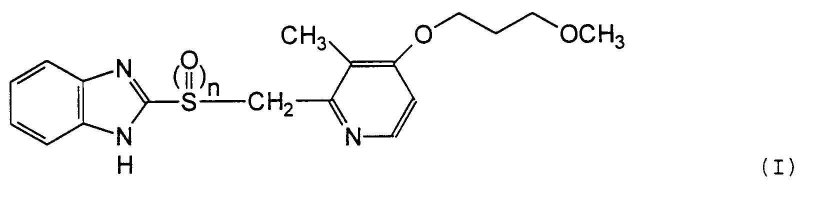 wherein n is 0 or 1, or a pharmacologically acceptable salt thereof for the preparation of a medicament for preventing, improving or treating a disease caused by Campylobacter pylori in a mammal suffering from such a disease.

2. The use as claimed in Claim 1, wherein the mammal is a human being.

3. The use as claimed in Claim 1, wherein the disease is gastritis.

4. The use as claimed in Claim 3, in which the gastritis is recurrent.

5. The use as claimed in Claim 1, wherein the disease is diarrhea.

6. The use as claimed in Claim 1, wherein the disease is food poisoning.

7. The use of a pharmacologically effective amount of a compound having the formula (I) as defined in claim 1 or a pharmacologically acceptable salt thereof for the preparation of a medicament for eradicating Campylobacter pylori from the mucous membrane of the stomach of a patient suffering from a gastric ulcer, duodenal ulcer or gastritis.

8. The use of a pharmacologically effective amount of a compound having the formula (I) as defined in claim 1 or a pharmacologically acceptable salt thereof for the preparation of a medicament for preventing recurrence of a disease caused by Campylobacter pylori.

9. The use of a pharmacologically effective amount of a compound having the formula (I) as defined in claim 1 or a pharmacologically acceptable salt thereof for the preparation of a medicament for restraining the adverse activity of C. pylori to cause a peptic disease.

10. The use according to any one of the preceding claims wherein the pharmaceutically active compound is employed as its sodium salt having the following formula (II): 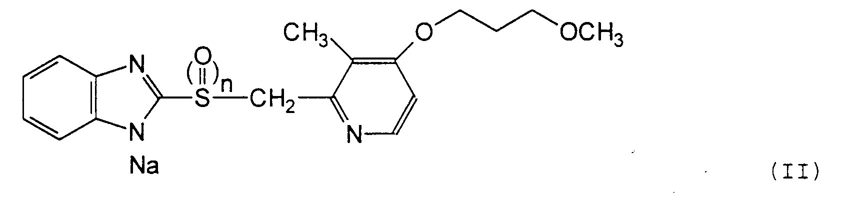 wherein n is 0 or 1.

11. The use according to any one of the preceding claims, wherein the disease is peptic ulcer.

## Patentansprüche

1. Verwendung einer pharmakologisch wirksamen Menge einer Verbindung mit der folgenden Formel (I) 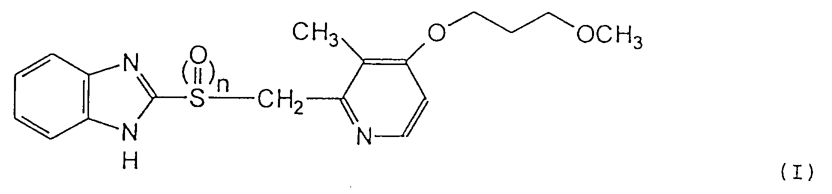 worin n 0 oder 1 ist, oder eines pharmakologisch annehmbaren Salzes davon zur Herstellung eines Arzneimittels zur Verhinderung, Verbesserung oder Behandlung einer Krankheit, die durch Campylobacter pylori verursacht wird, in einem Säugetier, das an einer solchen Krankheit leidet.

2. Verwendung gemäß Anspruch 1, wobei das Säugetier ein Mensch ist.

3. Verwendung gemäß Anspruch 1, wobei die Krankheit Gastritis ist.

4. Verwendung gemäß Anspruch 3, wobei die Gastritis rezidivierend ist.

5. Verwendung gemäß Anspruch 1, wobei die Krankheit Diarrhöe ist.

6. Verwendung gemäß Anspruch 1, wobei die Krankheit Lebensmittelvergiftung ist.

7. Verwendung einer pharmakologisch wirksamen Menge einer Verbindung mit der Formel (I) gemaß Anspruch 1 oder eines pharmakologisch annehmbaren Salzes davon zur Herstellung eines Medikaments zur Eradizierung von Campylobacter pylori von der Magenschleimhaut eines Patienten, der an einem Magengeschwür, Duodenalgeschwür oder Gastritis leidet.

8. Verwendung einer pharmakologisch wirksamen Menge einer Verbindung mit der Formel (I) gemäß Anspruch 1 oder eines pharmakologisch annehmbaren Salzes davon, zur Herstellung eines Medikaments zur Verhinderung eines Rezidivs einer Krankheit, die durch Campylobacter pylori verursacht wird.

9. Verwendung einer pharmakologisch wirksamen Menge einer Verbindung mit der Formel (I) gemäß Anspruch 1 oder eines pharmakologisch annehmbaren Salzes davon zur Herstellung eines Medikaments zur Unterdrückung der abträglichen Wirkung von C. pylori, eine peptische Krankheit zu verursachen.

10. Verwendung gemäß einem der vorhergehenden Ansprüche, wobei die pharmazeutisch wirksame Verbindung als ihr Natriumsalz mit der folgenden Formel (II) eingesetzt wird: 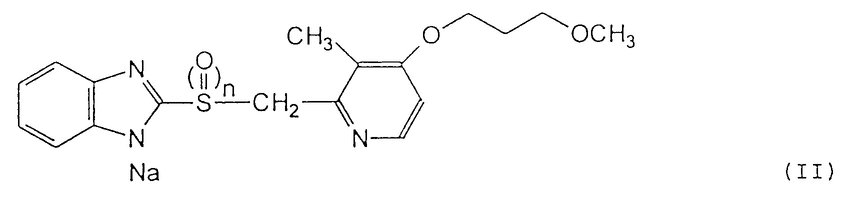 worin n 0 oder 1 ist.

11. Verwendung gemaß einem der vorhergehenden Ansprüche, wobei die Krankheit ein peptisches Ulcer ist.

## Revendications

1. Utilisation d'une quantité pharmacologiquement efficace d'un composé ayant la formule (I) suivante : 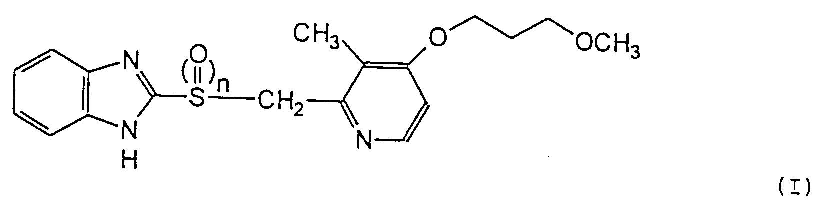 dans laquelle n est 0 ou 1,
ou d'un sel pharmacologiquement acceptable de celui-ci pour la préparation d'un médicament destiné à empêcher, enrayer ou traiter une maladie provoquée par *Campylobacter pylori* chez un mammifère souffrant d'une telle maladie.

2. Utilisation selon la revendication 1, dans laquelle le mammifère est un être humain.

3. Utilisation selon la revendication 1, dans laquelle la maladie est une gastrite.

4. Utilisation selon la revendication 3, dans laquelle la gastrite est récurrente.

5. Utilisation selon la revendication 1, dans laquelle la maladie est une diarrhée.

6. Utilisation selon la revendication 1, dans laquelle la maladie est une intoxication alimentaire.

7. Utilisation d'une quantité pharmacologiquement efficace d'un composé ayant la formule (I) telle que définie dans la revendication 1, ou d'un sel pharmacologiquement acceptable de celui-ci, pour la préparation d'un médicament destiné à éliminer des *Campylobacter pylor*i de la membrane muqueuse de l'estomac d'un patient souffrant d'un ulcère gastrique, d'un ulcère duodénal ou d'une gastrite.

8. Utilisation d'une quantité pharmacologiquement efficace d'un composé ayant la formule (I) telle que définie dans la revendication 1, ou d'un sel pharmacologiquement acceptable de celui-ci, pour la préparation d'un médicament destiné à empêcher la récurrence d'une maladie causée par *Campylobacter pylori*.

9. Utilisation d'une quantité pharmacologiquement efficace d'un composé ayant la formule (I) telle que définie dans la revendication 1, ou d'un sel pharmacologiquement acceptable de celui-ci, pour la préparation d'un médicament destiné à restreindre l'activité néfaste de *Campylobacter pylori* causant une maladie gastrique.

10. Utilisation selon l'une quelconque des revendications précédentes, dans laquelle le composé pharmaceutiquement actif est employé sous forme de son sel de sodium ayant la formule (II) suivante : 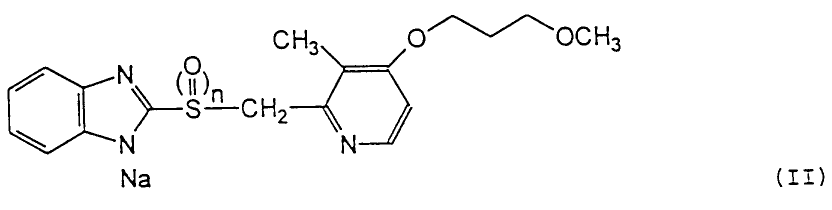 dans laquelle n est 0 ou 1.

11. Utilisation selon l'une quelconque des revendications précédentes, dans laquelle la maladie est un ulcère simple de l'estomac.
